(19) Europäisches Patentamt
European Patent Office
Office européen des brevets

(11) Veröffentlichungsnummer: **0 200 943 B1**

# (12) EUROPÄISCHE PATENTSCHRIFT

(45) Veröffentlichungstag der Patentschrift: **07.12.88**

(21) Anmeldenummer: **86104876.7**

(22) Anmeldetag: **09.04.86**

(51) Int. Cl.⁴: **C 07 C 149/32**, C 07 C 147/06, C 07 C 147/14, C 07 C 93/26, C 07 C 87/60, C 07 C 148/00, A 01 N 53/00

## (54) Vinylcyclopropancarbonsäureester.

(30) Priorität: **18.04.85 DE 3513978**

(43) Veröffentlichungstag der Anmeldung: **12.11.86 Patentblatt 86/46**

(45) Bekanntmachung des Hinweises auf die Patenterteilung: **07.12.88 Patentblatt 88/49**

(84) Benannte Vertragsstaaten: **AT BE CH DE FR GB IT LI NL SE**

(56) Entgegenhaltungen:
**EP-A- 0 031 199**
**EP-A- 0 054 360**
**EP-A- 0 060 617**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Naumann, Klaus, Dr., Richard-Wagner-Strasse 9, D-5090 Leverkusen (DE)**
Erfinder: **Braden, Rudolf, Dr., Nothauserfeld 1, D-5068 Odenthal (DE)**
Erfinder: **Behrenz, Wolfgang, Dr., Untergruendemich 14, D-5063 Overath (DE)**
Erfinder: **Becker, Benedikt, Dr., Metzkausener Strasse 14, D-4020 Mettmann (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

**EP 0 200 943 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft neue Vinylcyclopropancarbonsäureester, Verfahren zu ihrer Herstellung, ihre Verwendung als Pflanzenschutzmittel zur Bekämpfung von Hygiene- und Vorratsschädlingen sowie im Materialschutz, insbesondere als Insektizide und Akarizide sowie neue Zwischenprodukte zu ihrer Herstellung und Verfahren zu deren Herstellung.

Es ist bereits bekannt geworden, dass ähnlich strukturierte Cyclopropancarbonsäureester (z. B. aus EP-A 0060617) als Insektizide einsetzbar sind. Diese zeigen jedoch eine erheblich schwächer ausgeprägte Wirksamkeit als die erfindungsgemässen Verbindungen. Als naheliegende Vergleichsverbindung ist die Verbindung Nr. 2 aus EP-A 31199, Seite 4 aufzufassen. Auch gegenüber dieser Verbindung zeigen die erfindungsgemässen Wirkstoffe im überraschenden Masse überlegene biologische Eigenschaften.

Es wurden neue Vinylcyclopropancarbonsäureester der Formel I

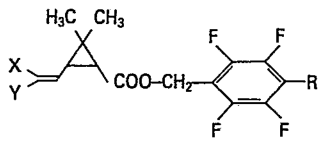 (I)

gefunden, in welcher R für Alkylthio($C_1$–$C_6$), Alkylsulfinyl($C_1$–$C_4$), Alkylsulfonyl ($C_1$–$C_6$), Amino, Monoalkyl-($C_1$–$C_6$)-amino oder Dialkyl-($C_1$–$C_6$)-amino steht und wobei X und Y entweder gleichzeitig für Halogen stehen oder X und Y gleichzeitig für Alkyl($C_1$–$C_4$) stehen, oder in welcher X für $CF_3$, Y für Cl und gleichzeitig R für Methylmercapto steht.

Man erhält die neuen Vinylcyclopropancarbonsäureester der Formel I,

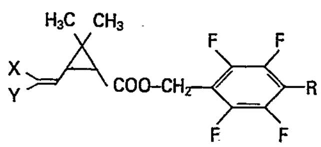 (I)

in welcher R für Alkylthio ($C_1$–$C_6$), Alkylsulfinyl ($C_1$–$C_4$), Alkylsulfonyl ($C_1$–$C_6$), Amino, Monoalkyl-($C_1$–$C_6$)-amino onder Dialkyl-($C_1$–$C_6$)amino steht und wobei X und Y entweder gleichzeitig für Halogen stehen oder X und Y gleichzeitig für Alkyl($C_1$–$C_4$) stehen, oder in welcher X für $CF_3$, Y für Cl und gleichzeitig R für Methylmercapto steht, wenn man eine Säure oder deren reaktionsfähiges Derivat der Formel II

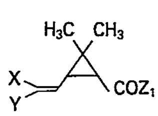 (II)

in welcher X und Y die oben angegebene Bedeutung besitzen und $Z_1$ Halogen, vorzugsweise Chlor oder OH, bedeutet, mit einem Alkohol oder dessen reaktionsfähigem Derivat der Formel III

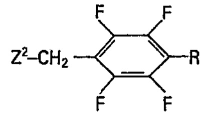 (III)

in welcher R die oben angegebene Bedeutung hat und $Z^2$ für OH, Cl oder Br steht, gegebenenfalls in Gegenwart von Lösungsmitteln, Säureakzeptoren und/oder Phasentransferkatalysatoren umgesetzt.

Vorzugsweise erfolgt die Umsetzung der Verbindungen (II) mit solchen der Formel (III) ohne Anwesenheit von Lösungsmitteln. Die Alkohole bzw. reaktionsfähigen Alkoholderivate besitzen folgende Formel:

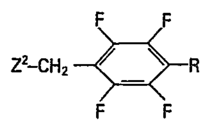 (III)

wobei R und $Z^2$ die oben angegebenen Bedeutungen besitzen.

Die Alkohole bzw. Alkoholderivate der Formel (III) kann man herstellen, indem man Pentafluorbenzylalkohol oder seine Derivate der Formel (IV)

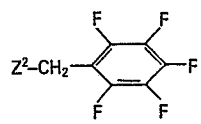 (IV)

wobei $Z^2$ für OH, Cl oder Br steht, gegebenenfalls in Anwesenheit von Säureakzeptoren mit Verbindungen der Formel (V)

R–H (V)

umsetzt, wobei R für Alkylthio ($C_1$–$C_6$), Alkylsulfinyl ($C_1$–$C_4$), Alkylsulfonyl ($C_{1–6}$), Amino, Monoalkyl-($C_1$–$C_6$)-amino oder Dialkyl-($C_1$–$C_6$)-amino steht.

Eine weitere Möglichkeit zur Herstellung von Verbindungen der Formel (I), in welcher der Rest R für Alkylthio ($C_1$–$C_6$) steht, besteht darin, dass man Verbindungen der Formel

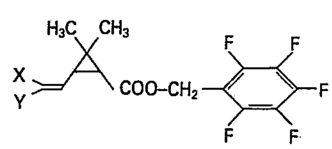 (VI)

in welcher X und Y die oben genannte Bedeutung besitzen mit Alkalialkylmercaptiden (vorzugsweise mit 1 bis 6, insbesondere 1–4 C-Atomen umsetzt, wobei, falls X für $CF_3$ und Y für Cl steht, nur mit Alkalimethylmercaptid umgesetzt wird. Vorzugsweise wird die Umsetzung von Permethrinsäurepentafluorbenzylester oder Chrysantemumsäurepentafluorbenzylester mit Natriumalkylmercaptid, insbesondere mit Natriummethylmercaptid im Zweiphasensystem (Wasser/nicht mit Wasser mischbares organisches Lösungsmittel wie z. B. Methylenchlorid) durchgeführt. Die Umsetzung wird bei 0 bis 80°C, vorzugsweise bei Raumtemperatur durchgeführt. Eine weitere bevorzugte Ausführungsform dieser Umsetzung erfolgt im System Toluol/Wasser/Phasentransferkatalysator oder auch in mit Wasser mischbaren niederen Alkoholen, wie z.B. Methanol, Ethanol oder Isopropanol. Die Aufarbeitung der organischen Phase geschieht jeweils in an sich bekannter Weise. Die Wirkstoffe werden vorzugsweise durch Destillation bei niedrigem Druck abgetrennt. Überraschenderweise zeigen die erfindungsgemässen Vinylcyclopropancarbonsäureester der Formel (I) eine erheblich höhere insektizide Wirkung als die aus dem Stand der Technik bekannten Verbindungen gemäss EP-A 0060617.

Die Verbindungen kommen in 4 stereoisomeren Formen vor, von denen die 2 mit der absoluten Konfiguration R am C-Atom, welches die Carboxygruppe trägt, in besonderer Weise Träger der insektiziden Wirkung sind.

Von den erfindungsgemässen Vinylcyclopropancarbonsäureestern der Formel (I) sind bevorzugt diejenigen, in denen R für Alkylthio ($C_1$–$C_6$), Alkylsulfonyl ($C_1$–$C_6$), Amino, Monoalkyl-($C_1$–$C_6$)-amino, Dialkyl-($C_1$–$C_6$)-amino steht und X und Y gleichzeitig für Chlor oder Brom stehen oder X und Y gleichzeitig für Alkyl ($C_1$–$C_4$) stehen.

Ganz besonders bevorzugt sind diejenigen Vinylcyclopropancarbonsäureester der Formel (I), in welcher R = Alkylthio ($C_1$–$C_4$), Alkylsulfinyl ($C_1$–$C_4$), X und Y = gleichzeitig Chlor oder X und Y = gleichzeitig Methyl.

Verwendet man beispielsweise Permethrinsäurechlorid und 2,3,5,6-Tetrafluor-4-methylmercaptobenzylalkohol als Ausgangskomponenten, so kann der Reaktionsverlauf durch das folgende Formelschema wiedergegeben werden:

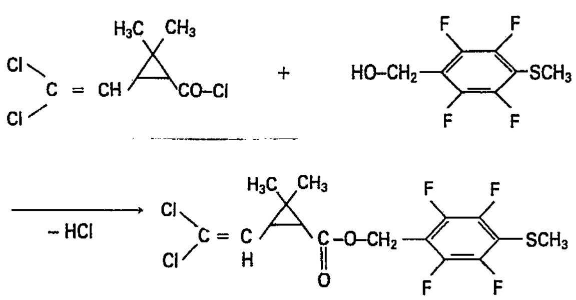


Die weitere Herstellungsvariante, Umsetzung von Pentafluorbenzylestern der Formel VI mit Alkalialkylmercaptiden, soll an dem folgenden Formelschema verdeutlicht werden:

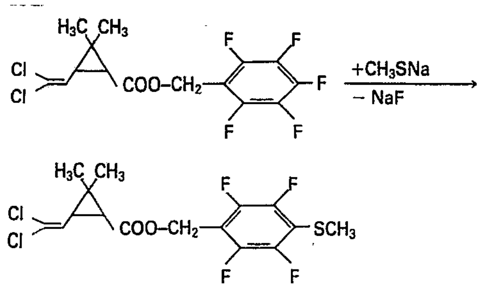


Die als Ausgangsprodukte zu verwendenden Vinylcyclopropancarbonsäuren bzw. deren Derivate der Formel (II) sind bekannt und nach allgemein üblichen in der Literatur beschriebenen Verfahren herstellbar (vgl. z.B. DE-OS 2326077, DE-OS 2802962 und US-PS 4236026).

Als Beispiele für die als Ausgangsprodukte zu verwendenden Verbindungen der Formel (II) seien genannt:

3-(2′,2′-Dichlorvinyl)-2,2-dimethyl-cyclopropancarbonsäure (und -chlorid), 3-(2′,2′-Dibromvinyl)-2,2-dimethyl-cyclopropancarbonsäure (und -chlorid), 3-(2′,2′-Dimethylvinyl)-2,2-dimethyl-cyclopropancarbonsäure (und -chlorid), 1-R-trans-3-(2,2-Dichlorvinyl)-2,2-dimethyl-cyclopropancarbonsäure (und -chlorid), 1-R-cis-3-(2,2-Dichlorvinyl)-2,2-dimethyl-cyclopropancarbonsäure (und -chlorid).

Die als Ausgangsprodukte zu verwendenden Benzylalkohole bzw. deren reaktionsfähigen Derivate der Formel (III) sind z.T. neu. Als reaktionsfähige Derivate werden bevorzugt die Chloride eingesetzt.

Als Beispiele für die als Ausgangsprodukte zu verwendenden Verbindungen der Formel III seien im einzelnen genannt:

4-Methylmercapto-2,3,5,6-tetrafluorbenzylalkohol, 4-Propyl-mercapto-2,3,5,6-tetrafluorbenzylalkohol, 4-Ethylmercapto-2,3,5,6-tetrafluorbenzylalkohol, 4-Butylmercapto-2,3,5,6-tetrafluorbenzylalkohol, 4-Dimethylamino-2,3,5,6-tetrafluorbenzylalkohol.

Die Umsetzung der Säuren bzw. reaktionsfähigen Derivate der Säuren der Formel (II) mit den Alkoholen oder den reaktionsfähigen Derivaten der Alkohole (III) geschieht vorzugsweise in Abwesenheit von Lösungsmitteln. Insbesondere werden so die Säurechloride (Formel II, $Z_1$ = Cl) umgesetzt, wobei man dann bis zum Ende der Chlorwasserstoffentwicklung erwärmt. Natürlich können auf diese Art auch andere Säurehalogenide wie z.B. Säurebromiden umgesetzt werden.

Die Aufarbeitung der Reaktionsprodukte erfolgt im allgemeinen durch Destillation.

Zur Herstellung der erfindungsgemässen Verbindungen der Formel I gemäss 1. (oben) aus Carbonsäuren bzw. Carbonsäurehalogeniden der Formel II und Alkoholen bzw. Chloriden oder Bromiden der Formel III können aber auch als Säureakzeptoren, z.B. alle üblichen Säurebindemittel, Verwendung finden.

Besonders bewährt haben sich Alkalihydroxide, -carbonate und -alkoholate, wie Kaliumhydroxid, Natriumhydroxid, Natriummethylat, Kaliumcarbonat, Natriumethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur der Umsetzung von Verbindungen (II) mit Verbindungen (III) kann innerhalb eines grösseren Bereiches variiert werden. Im allgemeinen arbeitet man bei der Umsetzung der Säurehalogenide mit Alkoholen zwischen 0 und 100°C, vorzugsweise bei 15 bis 40°C und bei der Umsetzung der Carbonsäure mit den Halogeniden zwischen 50 und 150°C, vorzugsweise bei 80 bis 120°C. Im letzteren Fall wird bevorzugt in Gegenwart eines Katalysators gearbeitet.

Als Katalysatoren kommen alle sogenannten Phasentransferkatalysatoren in Betracht, wie beispielsweise Kronenether oder quartäre Ammonium- oder Phosphoniumsalze. Bevorzugt sind quartäre Ammoniumsalze, wie beispielsweise Tetrabutylammoniumchlorid, Tetrabutylammoniumbromid, Benzyltriethylammoniumchlorid oder Methyltrioctylammoniumchlorid.

Die Umsetzung lässt man im allgemeinen bei Normaldruck ablaufen. Das Verfahren zur Herstellung der erfindungsgemässen Verbindungen wird bevorzugt ohne Mitverwendung von Lösungsmitteln durchgeführt. Selbstverständlich kann die Reaktion auch in Gegenwart geeigneter Lösungs- und Verdünnungsmittel durchgeführt werden. Als solche kommen praktisch alle inerten organischen Solventien in Frage. Hierzu gehören insbesondere aliphatische und aromatische, gegebenenfalls chlorierte Kohlenwasserstoffe wie Benzol, Toluol, Xylol, Benzin, Methylenchlorid, Chloroform, Dichlorethan, Chlorbenzol, o-Dichlorbenzol oder Ether, z. B. Diethyl-, Diisopropyl- oder Dibutylether, ausserdem Nitrile, wie Aceto- und Propionitril.

Eine weitere bevorzugte Herstellungsmethode ist die Umsetzung der Alkalisalze der Säuren mit entsprechenden Benzylhalogeniden der Formel III ($Z^2$ = Cl, Br) in Gegenwart z. B. von katalytischen Mengen Pentamethylethylentriamin o. ä. Aminen und beispielsweise in Acetonitril wie z. B. in Synthesis 1975, 805, beschrieben.

Zur Durchführung des Verfahrens setzt man die Ausgangskomponenten vorzugsweise in äquimolaren Mengen ein. Die Reaktionskomponenten werden gegebenenfalls in einem der angegebenen Lösungsmittel zusammengegeben und meist bei erhöhter Temperatur nach Zugabe des Säureakzeptors und gegebenenfalls des Katalysators zur Vervollständigung der Reaktion eine oder mehrere Stunden gerührt. Anschliessend giesst man das Reaktionsgemisch in Wasser, trennt die organische Phase ab und wäscht diese mit Wasser neutral. Nach dem Trocknen wird das Lösungsmittel im Vakuum abdestilliert.

Die Wirkstoffe eignen sich bei guter Pflanzenverträglichkeit und günstiger Warmblüteroxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber. Aus der Ordnung der Diplopoda z. B. Blaniulus guttulatus. Aus der Ordnung der Chilopoda z. B. Geophilus carpophagus, Scutigera spec. Aus der Ordnung der Symphyla z. B. Scutigerella immaculata. Aus der Ordnung der Thysanura z. B. Lepisma saccharina. Aus der Ordnung der Collembola z. B. Onychiurus armatus. Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria. Aus der Ordnung der Dermaptera z. B. Forficula auricularia. Aus der Ordnung der Isoptera z. B. Reticulitermes spp. Aus der Ordnung der Anoplura z. B. Phylloxera vastatrix, Pemphigus spp., Pediculus humanus corporis, Haematopinus spp., Linognathus spp. Aus der Ordnung der Mallophaga z. B. Trichodectes spp., Damalinea spp. Aus der Ordnung der Thysanoptera z. B. Hercinothrips femoralis, Thrips tabaci. Aus der Ordnung der Heteroptera z. B. Eurygaster spp., Dysdercus intermedius, Piesma quadrata, Cimex lectularius, Rhodnius prolixus, Triatoma spp. Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp. Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana. Aus der Ordnung der Coleoptera z. B. Anobium punctatum, Rhizopertha dominica, Bruchidius obtectus, Acanthoscelides obtectus, Hylotrupes bajulus, Agelastica alni, Leptionotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica. Aus der Ordnung der Hymenoptera z. B. Diprion spp., Hopplocampa spp., Lasius spp., Monomorium pharaonis, Vespa spp. Aus der Ordnung der Diptera z. B. Ädes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp.,

Tabanus spp., Tannia spp., Bibio hortulanus, Oscinella frit, Phorbia spp., Pegomyia hyoscyami, Ceratitis capitata, Dacus oleae, Tipula paludosa. Aus der Ordnung der Siphonaptera z. B. Xenopsylla cheopis, Ceratophyllus spp. Aus der Ordnung der Arachnida z. B. Scorpio maurus, Latrodectus mactans. Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV, Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweisshydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,05 und 50%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepassten üblichen Weise.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

Beispiel A

$LD_{100}$-Test. Testtiere: Blatta orientalis ♀♀; Lösungsmittel: Aceton.

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^3$ Filterpapier verschieden hoch. Anschliessend gibt man 5 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

Bei diesem Test zeigen z. B. die folgenden Verbindungen der Herstellungsbeispiele überlegene Wirksamkeit gegenüber dem Stand der Technik: 1, 2, 3, 4, 7, 10.

Beispiel B

$LT_{100}$-Test für Dipteren. Testtiere: Aedes aegypti; Lösungsmittel: Aceton.

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^3$ Filterpapier verschieden hoch. Anschliessend gibt man etwa 25 Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z. B. die folgenden Verbindungen der Herstellungsbeispiele überlegene Wirksamkeit gegenüber dem Stand der Technik: 1, 2, 4.

Beispiel C

Laphygma-Test. Lösungsmittel: 3 Gewichtsteile Dimethylformamid; Emulgator: 1 Gewichtsteil Alkylarylpolyglykolether.

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z. B. die folgenden Verbindungen der Herstellungsbeispiele überlegene Wirksamkeit gegenüber dem Stand der Technik: 1, 2, 3, 4.

Beispiel D

Nephotettix-Test. Lösungsmittel: 7 Gewichtsteile Dimethylformamid; Emulgator: 1 Gewichtsteil Alkylarylpolyglykolether.

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100%, dass alle Zikaden abgetötet wurden; 0% bedeutet, dass keine Zikaden abgetötet wurden.

Bei diesem Test zeigen z. B. die folgenden Verbindungen der Herstellungsbeispiele überlegene Wirksamkeit gegenüber dem Stand der Technik: 1, 2, 4, 19.

Beispiel E

Grenzkonzentrations-Test/Bodeninsekten. Testinsekt: Phorbia antiqua-Maden (im Boden); Lösungsmittel: 3 Gewichtsteile Aceton; Emulgator: 1 Gewichtsteil Alkylarylpolyglykolether.

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und lässt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z. B. die folgenden Verbindungen der Herstellungsbeispiele überlegene Wirksamkeit gegenüber dem Stand der Technik: 1, 2, 3, 4, 7, 10.

Beispiel F

Grenzkonzentrations-Test/Bodeninsekten. Testinsekt: Diabrotica balteata – Larven im Boden; Lösungsmittel: 3 Gewichtsteile Aceton; Emulgator: 1 Gewichtsteil Alkylarylpolyglykolether.

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in 0,5 l-Töpfe und lässt diese bei 20°C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z. B. die folgenden Verbindungen der Herstellungsbeispiele überlegene Wirksamkeit gegenüber dem Stand der Technik: 1, 2, 3, 4, 7, 10.

Beispiel G

$LT_{100}$-Test für Dipteren. Testtiere: Musca domestica; Lösungsmittel: Aceton.

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^3$ Filterpapier verschieden hoch. Anschliessend gibt man etwa 25 Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Bei diesem Test zeigen z. B. die folgenden Verbindungen der Herstellungsbeispiele überlegene Wirksamkeit gegenüber dem Stand der Technik: 1, 2, 4, 19.

Beispiel 1

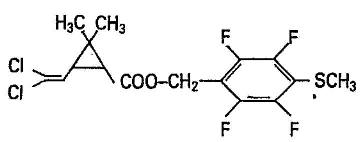

1-R-trans 0,1 Mol (20,3 g) 1 R trans-Permethrinsäurechlorid und 0,1 Mol (22,7 g) 2,3,5,6-Tetrafluor-4-methylmercaptobenzylalkohol wurden zusammen ohne Lösungsmittel auf 50 bis 70°C bis zum Ende der Chlorwasserstoffentwicklung erwärmt. Das Produkt wurde anschliessend im Vakuum destilliert. Man erhielt 39 g optisch aktive 1R trans Verbindung der obenstehenden Formel (Fp.: 53–54°C; IR-Spektrum: 3040, 2960, 2940, 2880, 1730, 1635, 1615, 1470, 1425, 1395, 1385, 1345, 1280, 1230, 1150–1180, 1115, 1050, 990, 970, 930, 910, 885, 860, 780.

In analoger Weise wurden die folgenden Verbindungen hergestellt:

allgemeine Formel:

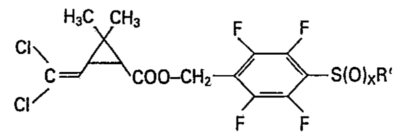

| Bsp. Nr. | R' | X | Stereochemie | Physikal. Daten |
|---|---|---|---|---|
| 2 | $CH_3$ | 0 | racem. cis/trans | Kp 0,1: 145°C |
| 3 | $CH_3$ | 0 | opt. akt. 1R cis | Fp.: 52°C |
| 4 | $CH_3$ | 0 | opt. akt. 1R cis/1R trans | Kp 0,1: 145°C |
| 5 | $C_2H_5$ | 0 | opt. akt. 1R trans | Fp.: 87°C |
| 6 | $nC_4H_9$ | 0 | opt. akt 1R trans | Kp 0,1: 165°C |
| 7 | $CH_3$ | 1 | racem. cis/trans | |
| 8 | $C_2H_5$ | 1 | | |
| 9 | $CH_3$ | 2 | | |

Beispiel 10

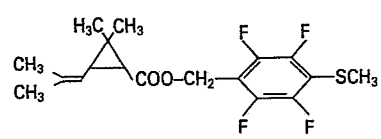

In analoger Weise zu Beispiel 1 wurden unter Einsatz von 0,1 Mol (±) cis/trans Chrysanthemumsäurechlorid und 0,1 Mol 2,3,5,6-Tetrafluor-4-methylmercaptobenzylalkohol 37 g des (±) cis/trans-Gemisches der oben aufgeführten Verbindung erhalten.

IR-Daten: 2930–3000, 2880, 1730, 1640, 1470, 1425, 1380, 1360, 1325, 1275, 1240, 1200, 1110–1170, 1050, 1020, 990, 930, 910, 855, 780.

In analoger Weise zu Beispiel 10 sind herstellbar:

allgemeine Formel:

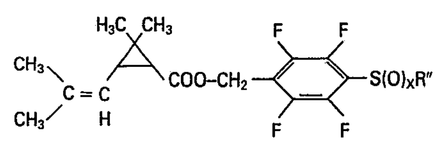

| Bsp. Nr. | X | R'' | Stereochemie |
|---|---|---|---|
| 11 | 0 | $CH_3$ | (±) cis/trans |
| 12 | 0 | $CH_3$ | opt. akt. 1R cis |
| 13 | 0 | $CH_3$ | opt. akt. 1R trans |
| 14 | 0 | $C_2H_5$ | opt. akt. 1R trans |
| 15 | 0 | $nC_4H_9$ | opt. akt. 1R trans |
| 16 | 1 | $CH_3$ | |
| 17 | 1 | $C_2H_5$ | |
| 18 | 2 | $CH_3$ | |

Beispiel 19

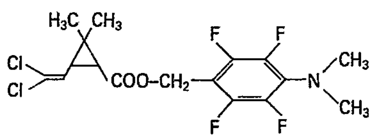

In Analogie zu Beispiel 1 wurden 0,1 Mol (22,3 g) 1R-trans-Permethrinsäurechlorid und 0,1 Mol (22,3 g) 2,2,5,6-Tetrafluor-4-dimethylaminobenzylalkohol umgesetzt.

Man erhielt 29 g des 1R-trans-Isomeren der o. g. Verbindung (Öl), IR-Daten: 2860–3000, 2820, 1730, 1650, 1515, 1490, 1440, 1430, 1385, 1345, 1280, 1260, 1230, 1170, 1100, 1050, 1000, 925, 895, 780, 750.

Beispiel 20

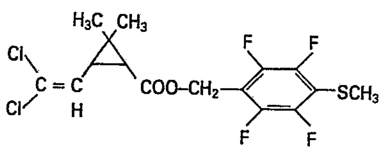

0,1 Mol (26 g) des Kaliumsalzes der Permethrinsäure wurden zusammen mit 0,1 Mol (24 g) 2,3,5,6-Tetrafluor-4-methylmercaptobenzylchlorid und 0,005 Mol (0,8 g) Pentamethyldiethylentriamin in 100 ml Acetonitril gegeben. Man erhitzt unter Rühren zum Rückfluss bis das 2,3,5,6-Tetrafluor-4-methylmercaptobenzylchlorid aufgebraucht worden ist, engt ein, schüttelt mit Wasser/Hexan aus. Die organische Lösung wird eingeengt und anschliessend wird im Hochvakuum destilliert. Schmelzpunkt: 54°C.

In analoger Weise zu Beispiel 20 können die Verbindungen der Beispiele 2 bis 19 zu den entsprechenden Pyrethroiden Endprodukten umgesetzt werden.

Beispiel 20a

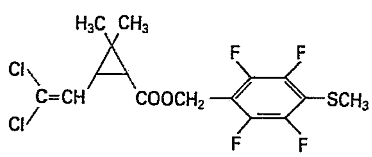

0,1 Mol (±) cis/trans Permethrinsäurepentafluorbenzylester in 150 ml Methylenchlorid wurde unter Stickstoff bei 20°C eine Lösung von 0,1 Mol Natriummethylmercaptid in 100 ml Wasser getropft. Nach Eintreten der Neutralreaktion wird die organische Phase eingeengt. Man erhält nach Destillation am Kugelrohr (230°C Ofentemperatur, 0,05 mm) die laut NMR-Spektrum reine Titelverbindung in 96 Prozent Ausbeute. Fp: 43–54°C.

In gleicher Weise erhält man aus 1R trans-Permethrinsäurepentafluorbenzylester den 1R trans-Permethrinsäuretetrafluor-4-methyl-mercaptobenzylester. Fp: 53–54°C.

Die vorgenannte Umsetzung kann auch im System Tuluol/Wasser/Phasentransferkatalysator oder auch z. B. in niedrigen Alkoholen wie Methanol, Ethanol oder Isopropanol durchgeführt werden.

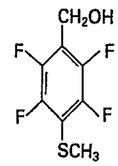

In einer 250 ml-Dreihalskolbenrührapparatur mit Thermometer, Kühler und Kühlbad werden 100 ml Isopropanol bei 0°C vorgelegt, dann werden 5 g Methylmercaptan eingeleitet und 4 g gepulvertes Natriumhydroxid zugegeben. Danach werden in 15 Minuten über einen beheizbaren Tropftrichter bei 0°C 20 g Pentalfluorbenzylalkohol zugetropft. Anschliessend wird langsam auf Rückflusstemperatur (83–84°C) erwärmt und eine Stunde bei dieser Temperatur gerührt. Der Ansatz wird abgekühlt und auf Eiswasser gegeben. Die entstehenden schmierigen Kristalle werden in Methylenchlorid aufgenommen.

Anschliessend werden die beiden Phasen getrennt, die organische Phase über Natriumsulfat getrocknet und an einer Kolonne destilliert. Man erhält 16,6 g 2,3,5-Tetrafluor-4-methylmercaptobenzylalkohol (Kp. 16 mbar: 145–146°C).

## Patentansprüche

1. Vinylcylclopropancarbonsäureester der Formel (I)

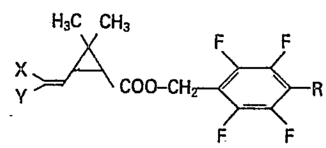 (I)

in welcher R für Alkylthio($C_1$–$C_6$), Alkylsulfinyl($C_1$–$C_4$), Alkylsulfonyl($C_1$–$C_6$), Amino, Monoalkyl-($C_1$–$C_6$)-amino oder Dialkyl-($C_1$–$C_6$)-amino steht und wobei X und Y entweder gleichzeitig für Halogen stehen oder X und Y gleichzeitig für Alkyl($C_1$–$C_4$) stehen, oder in welcher X für $CF_3$, Y für Cl und gleichzeitig R für Methylmercapto steht.

2. Vinylcyclopropancarbonsäureester der Formel (I) gemäss Anspruch 1, worin R = Alkylthio ($C_1$–$C_4$) oder Alkylsulfinyl ($C_1$–$C_4$) und wobei entweder X und Y gleichzeitig für Chlor oder gleichzeitig für Methyl stehen.

3. Vinylcyclopropancarbonsäureester gemäss Anspruch 1 der Formel

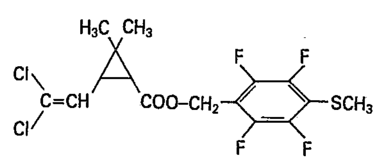

4. Verfahren zur Herstellung von Vinylcyclopropancarbonsäureestern der Formel (I)

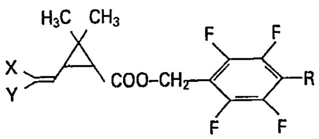 (I)

in welcher für Alkylthio ($C_1$–$C_6$), Alkylsulfinyl ($C_1$–$C_4$), Alkylsulfonyl ($C_1$–$C_6$), Amino, Monoalkyl-($C_1$–$C_6$)-amino oder Dialkyl-($C_1$–$C_6$)amino steht X und Y entweder gleichzeitig für Halogen stehen oder X und Y gleichzeitig für Alkyl ($C_1$–$C_4$) stehen, oder worin X für $CF_3$, Y für Cl und gleichzeitig R für Methylmercapto steht, dadurch gekennzeichnet, dass man eine Säure oder deren reaktionsfähiges Derivat der Formel II

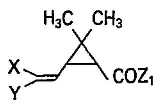 (II)

in welcher X und Y die oben angegebene Bedeutung besitzen und $Z_1$ Halogen, vorzugsweise Chlor oder OH, bedeutet, mit einem Alkohol oder dessen reaktionsfähigem Derivat der Formel III

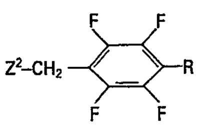 (III)

in welcher R die oben angegebene Bedeutung hat und $Z^2$ für OH, Cl oder Br steht, gegebenenfalls in Gegenwart von Lösungsmitteln, Säureakzeptoren und/oder Phasentransferkatalysatoren umsetzt.

5. Verfahren zur Herstellung von Vinylcyclopropancarbonsäureestern der Formel

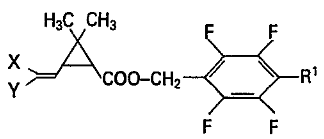 (Ia)

in welcher $R^1$ für Alkylthio-($C_1$–$C_6$) X und Y entweder gleichzeitig für Halogen stehen oder X und Y gleichzeitig für Alkyl($C_1$–$C_4$) stehen, oder worin X für $CF_3$, Y für Cl und gleichzeitig $R^1$ für Methylmercapto steht, dadurch gekennzeichnet, dass man einen Ester der Formel

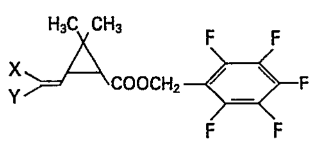 (IIa)

in welchem X und Y die oben angegebene Bedeutung besitzen, mit einem Alkalimercaptid der Formel

$[R^1]^{\ominus} Me^{\ominus}$ (IIb)

worin $R^1$ für den Alkyl-($C_1$–$C_4$)-mercaptic-Rest und $Me^+$ für ein Alkalimetallkation steht, in einem organischen Lösungsmittel oder in einem Zweiphasensystem Wasser/organisches Lösungsmittel umsetzt, wobei, falls in Formel IIa X für $CF_3$ und Y für Cl steht, der Rest $[R^1]^{\ominus}$ in Formel IIb ausschliesslich für das Methylmercaptid-Anion steht.

6. Schädlingsbekämpfungsmittel, gekennzeichnet durch einen Gehalt an mindestens einem Vinylcyclopropancarbonsäureester der Formel (I).

7. Verfahren zur Bekämpfung von tierischen Schädlingen, dadurch gekennzeichnet, dass man Vinylcyclopropancarbonsäureester der Formel (I) auf tierische Schädlinge und/oder ihren Lebensraum einwirken lässt.

8. Verwendung von Vinylcyclopropancarbonsäureestern der Formel (I) zur Bekämpfung von tierischen Schädlingen.

9. Verfahren zur Herstellung von Schädlingsbekämpfungsmitteln, dadurch gekennzeichnet, dass man Vinylcyclopropancarbonsäureester der Formel (I) mit Streckmitteln und/oder oberflächenaktiven Mitteln vermischt.

**Claims**

1. Vinylcyclopropanecarboxylic acid esters of the formula (I)

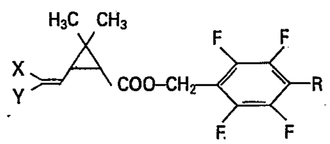 (I)

in which R represents alkylthio($C_1$–$C_6$), alkylsulphinyl($C_1$–$C_4$), alkylsulphonyl($C_1$–$C_6$), amino, monoalkyl-($C_1$–$C_6$)-amino or dialkyl-($C_1$–$C_6$)-amino, and wherein X and Y either simultaneously represent halogen or X and Y simultaneously represent alkyl($C_1$–$C_4$), or in which X represents $CF^3$, Y represents Cl and R simultaneously represents methylmercapto.

2. Vinylcyclopropanecarboxylic acid esters of the formula (I) according to Claim 1, wherein R = alkylthio ($C^1$–$C^4$) or alkylsulphinyl ($C^1$–$C^4$), and wherein X and Y either simultaneously represent chlorine or simultaneously represent methyl.

3. Vinylcyclopropanecarboxylic acid esters according to Claim 1 of the formula

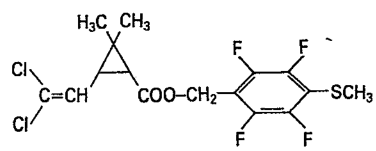

4. Process for the preparation of vinylcyclopropanecarboxylic acid esters of the formula (I)

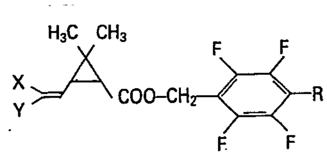 (I)

in which R represents alkylthio($C_1$–$C_6$), alkylsulphinyl($C_1$–$C_4$), alkylsulphonyl($C_1$–$C_6$), amino, monoalkylamino($C_1$–$C_6$) or dialkylamino($C_1$–$C_6$),

and wherein X and Y either simultaneously represent halogen or X and Y simultaneously represent alkyl($C_1$–$C_4$), or in which X represents $CF^3$, Y represents Cl and R simultaneously represents methylmercapto, characterised in that an acid, or a reactive derivative thereof, of the formula II

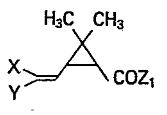

(II)

in which X and Y have the abovementioned meaning and $Z_1$ denotes halogen, preferably chlorine or OH, is reacted with an alcohol, or a reactive derivative thereof, of the formula III

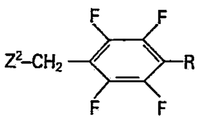

(III)

in which R has the abovementioned meaning and $Z^2$ represents OH, Cl or Br, if appropriate in the presence of solvents, acid acceptors and/or phase transfer catalysts.

5. Process for the preparation of vinylcyclopropanecarboxylic acid esters of the formula

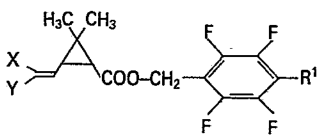

(Ia)

in which $R^1$ represents alkylthio($C_1$–$C_6$) and X and Y either simultaneously represent halogen or X and Y simultaneously represent alkyl($C_1$–$C_4$), or wherein X represents $CF_3$, Y represents Cl and $R^1$ simultaneously represents methylmercapto, characterised in that an ester of the formula

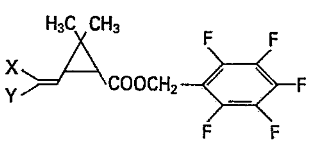

(IIa)

in which X and Y have the abovementioned meaning, is reacted with an alkali metal mercaptide of the formula

$[R^1]^{\ominus}$ Me $^{\ominus}$ (IIb)

wherein $R^1$ represents the alkylmercaptide radical and $Me^+$ represents an alkali metal cation, in an organic solvent or in a two-phase system of water/organic solvent, and wherein, if in formula IIa X represents $CF_3$ and Y represents Cl, the radical $[R^1]^{\ominus}$ in formula IIb exclusively represents the methylmercaptide anion.

6. Agents for combating pests, characterised in that they contain at least one vinylcyclopropanecarboxylic acid ester of the formula (I).

7. Method of combating animal pests, characterised in that vinylcyclopropanecarboxylic acid esters of the formula (I) are allowed to act on animal pests and/or their environment.

8. Use of vinylcyclopropanecarboxylic acid esters of the formula (I) for combating animal pests.

9. Process for the preparation of agents for combating pests, characterised in that vinylcyclopropanecarboxylic acid esters of the formula (I) are mixed with extenders and/or surface-active agents.

## Revendications

1. Ester d'acide vinylcyclopropane carboxylique, de formule (I)

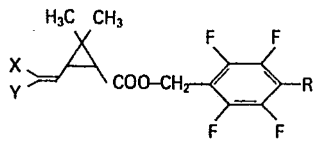

(I)

dans laquelle R représente un groupe alkylthio (en $C_1$–$C_6$), alkylsulfinyle (en $C_1$–$C_4$), alkylsulfonyle (en $C_1$–$C_6$), amino, monoalkyl (en $C_1$–$C_6$)-amino ou dialkyl(en $C_1$–$C_6$)-amino, et X et Y représentent simultanément chacun un atome d'halogène, ou bien X et Y représentent simultanément chacun un groupe alkyle (en $C_1$–$C_4$), ou bien X représente $CF_3$, Y représente Cl et, en même temps, R représente un groupe méthylmercapto.

2. Ester d'acide vinylcyclopropane carboxylique de formule (I), selon la revendication 1, dans lequel R représente un groupe alkylthio (en $C_1$–$C_4$) ou alkylsulfinyle (en $C_1$–$C_4$), et X et Y représentent simultanément chacun un atome de chlore ou simultanément chacun un groupe méthyle.

3. Ester d'acide vinylcyclopropane carboxylique selon la revendication 1, de formule

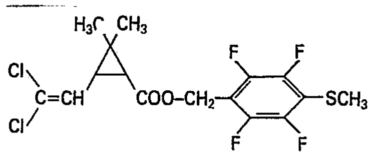

4. Procédé pour préparer des esters d'acides vinylcyclopropane carboxyliques de formule (I):

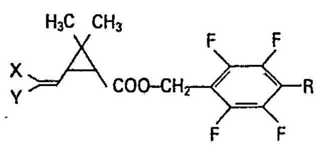

(I)

dans laquelle R représente un groupe alkylthio (en $C_1$–$C_6$), alkylsulfinyle (en $C_1$–$C_4$), alkylsulfonyle (en $C_1$–$C_6$), amino, monoalkyl (en $C_1$–$C_6$)-amino ou dialkyl (en $C_1$–$C_6$)-amino, X et Y représentent chacun, simultanément, un atome d'halogène, ou bien X et Y représentent chacun, simultanément, un groupe alkyle (en $C_1$–$C_4$), ou X représente $CF_3$, Y représente Cl, en même temps, R représente un groupe méthylmercapto, procédé caractérisé en ce qu'on fait réagir, éventuellement en présence de solvants, d'accepteurs d'acides et/ou de catalyseurs de transfert de phase, un acide, ou son dérivé réactif, de formule (II)

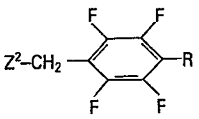

(III)

dans laquelle X et Y ont le sens indiqué ci-dessus, et $Z_1$ représente un halogène, avantageusement le chlore, ou OH, avec un alcool ou son dérivé réactif, de formule (III)

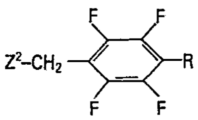

(III)

dans laquelle R a le sens indiqué ci-dessus et $Z^2$ représente –OH–, Cl ou Br.

5. Procédé pour préparer des esters d'acides vinylcyclopropane carboxyliques de formule

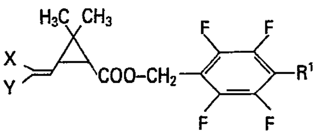

(Ia)

dans laquelle $R^1$ représente un groupe alkylthio (en $C_1$–$C_6$), X et Y représentent simultanément chacun un atome d'halogène, ou bien X et Y représentent simultanément chacun un groupe alkyle (en $C_1$–$C_4$), ou bien X représente $CF_3$, Y représente Cl et, en même temps, $R^1$ représente un groupe méthylmercapto, procédé caractérisé en ce qu'on fait réagir, dans un solvant organique ou dans un système à deux phases eau/solvant organique, un ester de formule

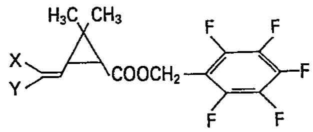

(IIa)

dans laquelle X et Y ont le sens indiqué ci-dessus, avec un mercaptide alcalin de formule

$[R^1]^{\ominus}Me^{\oplus}$ (IIb)

dans laquelle $R^1$ représente le reste alkyl (en $C_1$–$C_4$)-mercaptide, et $Me^+$ représente un cation de métal alcalin, et, si, dans la formule IIa, X représente $CF_3$ et Y représente Cl, le reste $[R^1]^{\ominus}$ représente exclusivement dans la formule IIb l'anion méthylmercaptide.

6. Produit pour la lutte contre les parasites, caractérisé en ce qu'il contient au moins un ester d'acide vinylcyclopropane carboxylique de formule (I).

7. Procédé pour combattre des parasites animaux, caractérisé en ce qu'on fait agir un ester d'acide vinylcyclopropane carboxylique, de formule (I), sur les parasites animaux et/ou sur leur biotope.

8. Utilisation d'esters d'acides vinylcyclopropane carboxyliques de formule (I), pour combattre des parasites animaux.

9. Procédé pour préparer des produits (pesticides) pour lutter contre les parasites, caractérisé en ce qu'on mélange un ester d'acide vinylcyclopropane carboxylique, de formule (I), avec des agents d'allongement et/ou avec des agents tensioactifs.